# EUROPÄISCHE PATENTANMELDUNG

(11) **EP 2 338 526 A1**
(43) Veröffentlichungstag der Anmeldung: **29.06.2011**
(21) Anmeldenummer: 09306195.0
(22) Anmeldetag: 08.12.2009
(51) Int. Cl.: A61L 9/01

(54) **Desodorierende Formulierung**

(71) Anmelder: Biothys S.A., 8399 Windhof (LU)
(72) Erfinder: Wuest, Robert, 67560, Rosheim (FR)
(74) Vertreter: Nuss, Laurent

(57) **Zusammenfassung**

Die Erfindung betrifft eine desodorierende Formulierung, insbesondere für eine Mischung aus Bitumen und Schwefel, die aktive, geruchsmindernde Agenzien auf Basis von Salicylsäureestern, Aldehyden und gegebenenfalls Alkoholen, sowie ein Verdünnungsmittel enthält. Außerdem betrifft sie Verfahren zur Herstellung einer desodorierenden Zusammensetzung auf Basis von Schwefel, die zur Geruchsminderung in Bitumen eingemischt werden kann.

## Beschreibung

Die Erfindung betrifft eine desodorierende Formulierung, insbesondere für eine Mischung aus Bitumen und Schwefel, die aktive, geruchsmindernde Agenzien und ein Verdünnungsmittel enthält, sowie Verfahren zur Herstellung einer Zusammensetzung auf Basis von Schwefel, die zur Geruchsverminderung eingesetzt werden kann.

Es ist bekannt, dass man durch Zusatz von untergeordneten Mengen an elementarem Schwefel die Eigenschaften von Bitumen verbessern kann, beispielsweise wird die Spurrillenbildung und die thermische Rissbildung vermindert und die Verarbeitung des Bitumens kann bei niedrigeren Temperaturen im Bereich von 130 bis 150°C vorgenommen werden.

Dabei kann es aber zu Geruchsbelästigungen insbesondere durch Schwefelwasserstoff und Schwefeldioxid kommen, die bei diesen Temperaturen gebildet werden. Daneben verdunsten bei diesen Temperaturen ein Großteil der im Bitumen enthaltenen flüchtigen Substanzen, z.B. organische Schwefel- und Stickstoffverbindungen, die ebenfalls einen üblen Geruch aufweisen.

Die US-A 3,960,585 und die WO 2005/059016 beschreiben, dass man die Bildung von Schwefelwasserstoff beim Erhitzen einer Mischung aus Asphalt und Schwefel durch Zusatz von freie Radikale bildenden Inhibitoren und /oder Redoxkatalysatoren unterdrücken kann. Diese Zusätze sind jedoch nicht ausreichend wirksam.

Der Erfindung lag daher die Aufgabe zugrunde, diese Geruchsbelästigung bei Mischungen von Bitumen und Schwefel zu unterdrücken oder wenigstens zu vermindern.

Diese Aufgabe wird durch eine desodorierende Formulierung nach Anspruch 1 gelöst, die aktive Agenzien und mindestens ein Verdünnungsmittel enthält, wobei die aktiven Agenzien folgende Komponenten umfassen:
- einen Ester der Salicylsäure,
- einen oder mehrere Aldehyde mit mindestens 6 Kohlenstoffatomen,
- gegebenenfalls einen oder mehrere Alkohole mit mindestens 6 Kohlenstoffatomen,
   sowie gegebenenfalls

- ein oder mehrere Terpene
- und/oder ein oder mehrere Ketone.

Vorzugsweise bestehen die aktiven Agenzien aus folgenden Komponenten:
A. 20 bis 60, vorzugsweise 30 bis 50 Gew.% Benzylsalicylat oder einem C1- bis C8- Alkylsalicylat,
B. 4 bis 30, vorzugsweise 5 bis 25 Gew.% eines C6- bis C12-Alkylaldehyds,
C. 0 bis 40, vorzugsweise 10 bis 30 Gew.% eines weiteren Aldehyds mit 8 bis 18 Kohlenstoffatomen,
D. 0 bis 40, vorzugsweise 10 bis 30 Gew.% eines Alkohols mit 8 bis 15 Kohlenstoffatomen,
E. 0 bis 5, vorzugsweise 0,5 bis 3 Gew.% eines Terpens und
F. 0 bis 4 Gew.% eines Ketons, wobei sich die Prozentzahlen auf 100 addieren.

In EP-A 1 235 768 und WO 2009/019 041 sind Verfahren zur Geruchsminderung von Bitumen beschrieben, bei denen in flüssiges Bitumen geruchsmindernde Formulierungen eingemischt werden, die insbesondere bestimmte Carbonsäureester bzw. bestimmte Aldehyde enthalten. Die übel riechenden flüchtigen Bestandteile von Bitumen bestehen im Wesentlichen aus organischen Schwefel- und Stickstoffverbindungen, während im vorliegenden Fall vor allem Schwefelwasserstoff und Schwefeldioxid beseitigt werden müssen.

Die Bitumen/Schwefel- Mischung enthält vorzugsweise untergeordnete Mengen an Schwefel, insbesondere 20 bis 40 Gew.%.

Die desodorierende Formulierung aus aktiven Agenzien und Verdünnungsmittel wird vorzugsweise in Mengen von 50 bis 1000 ppm, insbesondere von 100 bis 500 ppm, in die Bitumen/Schwefel- Mischung eingearbeitet, wobei die Formulierung vorzugsweise zu 5 bis 50 Gew.% und insbesondere zu 10 bis 40 Gew.% aus aktiven Agenzien und zu 95 bis 50 Gew.%, insbesondere zu 90 bis 60 Gew.% aus Verdünnungsmittel besteht.

Die Komponente A ist vorzugsweise Benzylsalicylat, daneben sind auch andere Alkylsalicylate mit 1 bis 8 Kohlenstoffatomen im Alkylrest geeignet, beispielsweise Methylsalicylat. Benzylsalicylat hat den großen Vorteil, dass es aufgrund seines sehr hohen Siedepunktes ein extrem niedrigen Dampfdruck hat, so dass die desodorierende Formulierung in der Mischung von Bitumen und Schwefel sehr lange, z.B. mehrere Monate lang wirksam bleibt.

Die Komponente B ist vorzugsweise Heptanal oder Dodecanal.

Die weiteren Aldehyde der Komponente C sind vorzugsweise ausgewählt aus:
Jasmonal (2- Benzylidenheptanal C₁₄H₁₈O)
Jasmonal H ((2E)-2-Benzylidenoctanal C₁₅H₂₀O)
Lilial (3-(4-tert.butylphenyl)butanal C₁₄H₂₀O)
Helional (3-benzo(1,3)dioxol-5-yl-2-methyl-propanal C₁₁H₁₂O₃)
Cyclamenaldehyd (3-(4-propan-2ylphenyl)butanal C₁₃H₁₈O)
2-Methylundecanal (C₁₂H₂₄O), sowie Mischungen davon.

Die Alkohole der Komponente D sind bevorzugt natürlich vorkommende Alkohole, ausgewählt aus:
Eugenol (2-methoxy-4-prop-2-enyl-phenol C₁₀H₁₂O₂)
Linalool (3,7-dimethylocta-1,6-dien-3-ol C₁₀H₁₈O)
Geraniol ((2E)-3,7-dimethylocta-2,6-dien-1-ol C₁₀H₁₈O)

Die optionalen Terpene der Komponente E sind vorzugsweise ausgewählt aus:
Alpha- Pinen (4,7,7-trimethylbicyclo(3.1.1)hept-3-en C₁₀H₁₆)
Alpha- Terpineol (2-(4-methyl-1-cyclohex-3-enyl)propan-2-ol C₁₀H₁₈O)
Limonen (1-methyl-4-prop-1-en-2-yl-cyclohexen C₁₀H₁₆).

Die optionalen Ketone der Komponente F sind vorzugsweise ausgewählt aus:
Benzophenon, Butyl-methyl-keton und 1,8-Cineol.

Die aktiven Agenzien werden zusammen mit einem Verdünnungsmittel angewandt.

Dieses bewirkt, dass die desodorierende Formulierung in flüssiger Form vorliegt. Gleichzeitig wirkt sie als Mischungsvermittler beim Vermischen der einzelnen Komponenten. Außerdem verhindert sie, dass bei höherer Temperatur einzelne relativ niedrig siedende Bestandteile der aktiven Agenzien verdampfen. Bevorzugte Verdünnungsmittel sind Mineralöle, d.h. Kohlenwasserstoffmitteldestillate, wie z.B. CATENEX der Fa. SHELL, sowie Pflanzenöle, wie Sonnenblumenöl oder Mischungen davon.

Die vorliegende Erfindung betrifft ferner ein Verfahren gemäß Anspruch 9.

Bei der Einarbeitung der erfindungsgemäßen desodorierenden Formulierung in die Bitumen/Schwefel- Mischung wird zunächst eine desodorierende Zusammensetzung auf Basis von Schwefel und 0,02 bis 0,3 Gew.% der desodorierenden Formulierung, gegebenenfalls zusammen mit 0 bis 8 Gew.% an weiteren Zusatzstoffen, jeweils bezogen auf Schwefel, hergestellt. Geeignete Zusatzstoffe sind Verarbeitungshilfsmittels, beispielsweise Ethylenbisstearylamid oder Amylacetat, und Farbmittel, beispielsweise Kohlenstoffpulver. Dieses Gemisch kann dann bei 140 bis 170°C in flüssiges Bitumen eingearbeitet werden.

"Auf Basis von Schwefel" bedeutet, dass Schwefel der wesentliche Bestandteil der Zusammensetzung ist.

Vorzugsweise werden dabei zunächst die desodorierende Formulierung, sowie gegebenenfalls das Verarbeitungshilfsmittel und das Farbmittel, bei 120 bis 150°C in geschmolzenen Schwefel eingemischt, das Gemisch wird pelletisiert und die Pellets werden dann in Bitumen eingearbeitet.

Es ist jedoch auch möglich, zunächst nur ein teilchenförmiges Gemisch aus Schwefel und gegebenenfalls dem Verarbeitungshilfsmittel und dem Farbmittel herzustellen und die desodorierende Formulierung auf die Teilchen oberflächlich aufzutragen, zum Beispiel nach in der Kunststofftechnologie üblichen Verfahren durch Besprühen, durch Auftrommeln oder durch Compoundieren.

### Beispiel 1:

Ein Gemisch von aktiven Agenzien wurde hergestellt aus:
40 Gew.teilen Benzylsalicylat,
18 Gew.teilen Dodecanal,
7 Gew.teilen Jasmonal H,
3 Gew.teilen Cyclamenaldehyd,
9 Gew.teilen Jasmonal,
10 Gew.teilen Linalool,
9 Gew.teilen Eugenol,
1 Gew.teil Limonen,
2 Gew.teilen Alpha- Terpineol, und
1 Gew.teil Alpha- Pinen.
30 Gew.teile dieser Mischung wurden mit 70 Gew.teilen CATENEX vermischt.

0,1 Gew.teile dieser flüssigen Formulierung wurden zusammen mit 3 Gew.teilen Ethylenbisstearylamid und 1 Gew.teil Kohlenstoffpulver bei 130°C in 100 Gew.teile flüssigen Schwefel eingemischt. Die Mischung wurde pelletisiert und die Pellets wurden bei 140°C im Gewichtsverhältnis 30 zu 70 mit flüssigem Bitumen vermischt. Dabei wurde die Bildung von H₂S und SO₂ weitgehend unterdrückt. Auch beim Auftrag der Bitumen/Schwefel- Mischung als Straßenbelag oder bei Herstellung von Dachpappe entstand nur eine geringe Geruchsbelästigung.

### Beispiel 2:

Ein Gemisch von aktiven Agenzien wurde hergestellt aus:
43 Gew.teilen Methylsalicylat,
11 Gew.teilen Heptanal,
10 Gew.teilen Jasmonal H,
5 Gew.teilen Lilial,
7 Gew.teilen Jasmonal,
5 Gew.teilen 2- Methylundecanal,
2 Gew.teilen Helional,
11 Gew.teilen Linalool,
6 Gew.teilen Geraniol,
30 Gew.teile dieser Mischung wurden mit 70 Gew.teilen CATENEX vermischt.

0,12 Gew.teile dieser Formulierung wurden zusammen mit 3,5 Gew.teilen Ethylenbisstearylamid in 100 Gew.teile flüssigen Schwefel bei 130°C eingemischt. Die Mischung wurde pelletisiert und die Pellets wurden wie in Beispiel 1 in flüssigen Bitumen eingemischt, wobei ebenfalls nur eine geringe Geruchsbelästigung entstand.

## Patentansprüche

1. Desodorierende Formulierung, insbesondere für eine Mischung aus Bitumen und Schwefel, aus aktiven Agenzien und mindestens einem Verdünnungsmittel, **dadurch gekennzeichnet, dass** die aktiven Agenzien folgende Komponenten umfassen:
- einen Ester der Salicylsäure,
- einen oder mehrere Aldehyde mit mindestens 6 Kohlenstoffatomen,
- gegebenenfalls einen oder mehrere Alkohole mit mindestens 6 Kohlenstoffatomen,
sowie gegebenenfalls
- ein oder mehrere Terpene
- und/oder ein oder mehrere Ketone.

2. Desodorierende Formulierung nach Anspruch 1, **dadurch gekennzeichnet, dass** die aktiven Agenzien aus folgenden Komponenten bestehen:
A. 20 bis 60 Gew.% Benzylsalicylat oder einem C1- bis C8-Alkylsalicylat,
B. 4 bis 30 Gew.% eines C6- bis C12- Alkylaldehyds,
C. 0 bis 40 Gew.% eines weiteren Aldehyds mit 8 bis 18 Kohlenstoffatomen,
D. 0 bis 40 Gew.% eines Alkohols mit 8 bis 15 Kohlenstoffatomen,
E. 0 bis 5 Gew.% eines Terpens, und
F. 0 bis 4 Gew.% eines Ketons,
wobei sich die Prozentzahlen auf 100 addieren.

3. Desodorierende Formulierung nach Anspruch 2, **dadurch gekennzeichnet, dass** die Komponente A Benzylsalicylat oder Methylsalicylat ist.

4. Desodorierende Formulierung nach den Ansprüchen 2 und 3, **dadurch gekennzeichnet, dass** die Komponente B Heptanal oder Dodecanal ist.

5. Desodorierende Formulierung nach den Ansprüchen 2 bis 4, **dadurch gekennzeichnet, dass** die Komponente C ausgewählt ist aus Jasmonal, Jasmonal H, Lilial, Helional, Cyclamenaldehyd, Methylundecanal oder Mischungen davon.

6. Desodorierende Formulierung nach den Ansprüchen 2 bis 5, **dadurch gekennzeichnet, dass** die Komponente D ausgewählt ist aus Eugenol, Linalool, Geraniol oder Mischungen davon.

7. Desodorierende Formulierung nach den Ansprüchen 2 bis 6, **dadurch gekennzeichnet, dass** die Komponente E ausgewählt ist aus Alpha-Pinen, Alpha- Terpineol, Limonen oder Mischungen davon.

8. Desodorierende Formulierung nach Anspruch 1, **dadurch gekennzeichnet, dass** sie 5 bis 50 Gew.% der aktiven Agenzien und 95 bis 50 Gew.% eines Verdünnungsmittel, vorzugsweise eines Mineralöls enthält.

9. Verfahren zur Herstellung einer desodorierenden Zusammensetzung auf Basis von Schwefel, **dadurch gekennzeichnet, dass** man in flüssigen Schwefel bei Temperaturen zwischen 120 und 150°C 0,02 bis 0,3 Gew.% der desodorierenden Formulierung nach Anspruch 1, sowie gegebenenfalls 0 bis 8 Gew.% weiterer Zusatzstoffe, jeweils bezogen auf Schwefel, einmischt.

10. Verfahren zur Herstellung einer desodorierenden Zusammensetzung auf Basis von Schwefel, **dadurch gekennzeichnet, dass** man auf teilchenförmigen Schwefel 0,02 bis 0,3 Gew.% der desodorierenden Formulierung nach Anspruch 1, sowie gegebenenfalls 0 bis 8 Gew.% weiterer Zusatzstoffe, jeweils bezogen auf Schwefel, oberflächlich aufbringt.
